# FASCICULE DE BREVET EUROPEEN

(11) **EP 2 349 113 B1**
(45) Date de publication et mention de la délivrance du brevet: **08.07.2015**
(21) Numéro de dépôt: 09737008.4
(22) Date de dépôt: 07.08.2009
(51) Int. Cl.: A61F 2/44

(54) **PROTHESE DE DISQUE INTERVERTEBRAL AUTO-ADAPTATIVE ET AUTO-STABLE**
SELBSTANPASSENDE UND SELBSTSTABILISIERENDE BANDSCHEIBENPROTHESE
SELF-ADJUSTING AND SELF-STABILIZING INTERVERTEBRAL DISC PROSTHESIS

(30) Priorité: 07.08.2008 FR 0804508
(43) Date de publication de la demande: 03.08.2011
(73) Titulaire: Lemaire, Valérie, 21910 Saulon La Chapelle (FR); Lavaste, François, 91240 Saint-Michel-sur-Orge (FR)
(72) Inventeur: LEMAIRE, Jean-Philippe, décédé (FR); LAVASTE, François, 91240 Saint-Michel-sur-Orge (FR)
(74) Mandataire: Oudin, Stéphane
(86) Numéro de dépôt international: PCT/FR2009/000992
(87) Numéro de publication internationale: WO 2010/015755

(56) Documents cités:
- WO-A-2005/094737
- WO-A-2006/105603
- US-A1- 2004 138 753
- US-A1- 2006 025 862
- US-A1- 2007 270 958

## Description

La présente invention concerne une prothèse de disque intervertébral destinée à être substituée aux disques fibro-cartilagineux assurant la liaison entre les vertèbres de la colonne vertébrale, et plus spécifiquement au niveau du rachis cervical.

Dans ce domaine, un grand nombre de prothèses antérieures ont déjà été réalisées. Toutefois, une partie de ces prothèses peuvent entraîner des effets indésirables chez les patients munis desdites prothèses. En effet, si elles sont réalisées en matériau trop compressible ou bien si elles permettent aux différentes pièces constituant lesdites prothèses d'avoir un mouvement trop important les unes par rapport aux autres, il y a un risque d'éjection d'au moins une partie de la prothèse vers l'extérieur des vertèbres. En outre, la littérature fait apparaître que la plupart de ces prothèses ne reproduisent pas correctement la cinématique de l'unité vertébrale avec un positionnement en cyphose segmentaire progressive expliquant les contraintes de charges exercées sur les articulations inter-apophysaires postérieures (couramment dénommées articulaires postérieures) entraînent un risque élevé de développement d'arthrose au niveau de ces articulaires postérieures. La principale difficulté est de réussir à contrôler les mouvements relatifs des différentes pièces constituant ces prothèses.

On rappellera que l'Unité Fonctionnelle Vertébrale (UFV) est un système complexe intégrant le disque intervertébral, les articulaires postérieures et le système musculo-ligamentaire. Ce système complexe ne possède pas de plan de symétrie transverse horizontal : en effet, au niveau anatomique, dans le plan sagittal, le contour de la surface inférieure de la vertèbre sus-jacente présente une forme concave en auge tandis que le contour de la surface supérieure de la vertèbre sous-jacente est globalement plan. Le creusement de la surface inférieure de la vertèbre sus-jacente est bien marqué au niveau des vertèbres cervicales, tandis qu'il est moins prononcé au niveau des lombaires. Le disque intervertébral compris entre ces deux surfaces ne présente donc pas de symétrie transverse horizontale.

Cette unité s'intégrant dans un ensemble, l'équilibre sagittal minimise les contraintes sur le Système Ligamentaire Postérieur, et par conséquent sur l'unité fonctionnelle, contribuant à définir la Zone Neutre (zone de mobilité ne mettant pas à contribution la résistance mécanique intrinsèque des différents éléments).

La pathologie dégénérative est le résultat d'une perte de la rigidité physiologique discale. La mobilité se résoud en un mouvement élémentaire, en particulier en translation transformant la fonction guide des articulaires en une fonction de butées.

Toute prothèse intervertébrale aura donc pour première contrainte non seulement de restaurer une amplitude de mobilité mais aussi de préserver ou restaurer la zone neutre, c'est-à-dire un ratio amplitude/zone neutre physiologique. Ce ratio fait intervenir la notion de cohérence des CIR (centre instantané de rotation) avec ceux de l'UFV.

La deuxième contrainte est le contrôle des couplages, c'est-à-dire contrôler le chargement articulaire, ce d'autant que l'on s'adresse à une articulation pathologique avec un système ligamentaire postérieur altéré, entraînant des modifications de l'équilibre sagittal, et participant à l'élargissement de la Zone Neutre. Il faut donc un freinage de la translation postéro-antérieure en flexion-extension, en diminuant le ratio translation-rotation, et en induisant un auto centrage par translation antéro-postérieure. Même principe pour l'inclinaison latérale avec une translation opposée induite par la rotation.

On connaît dans l'art antérieur des prothèses intervertébrales comprenant trois pièces dont un premier plateau dit plateau supérieur, un second plateau dit plateau inférieur et un noyau mobile disposé entre lesdits plateaux. Le noyau mobile de ces prothèses comporte une surface supérieure convexe de contact avec le plateau supérieur et une surface inférieure concave de contact avec le plateau inférieur. On trouvera des exemples de telles prothèses dans les documents WO 2005/094737, WO 2006/105603, US2007/270958 ou US 2004/138753.

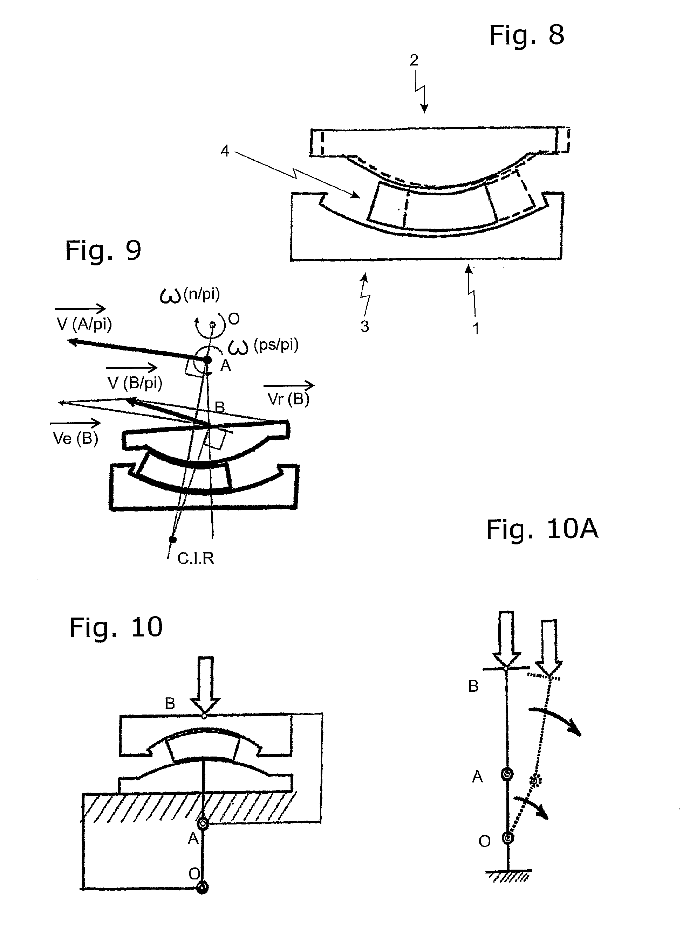

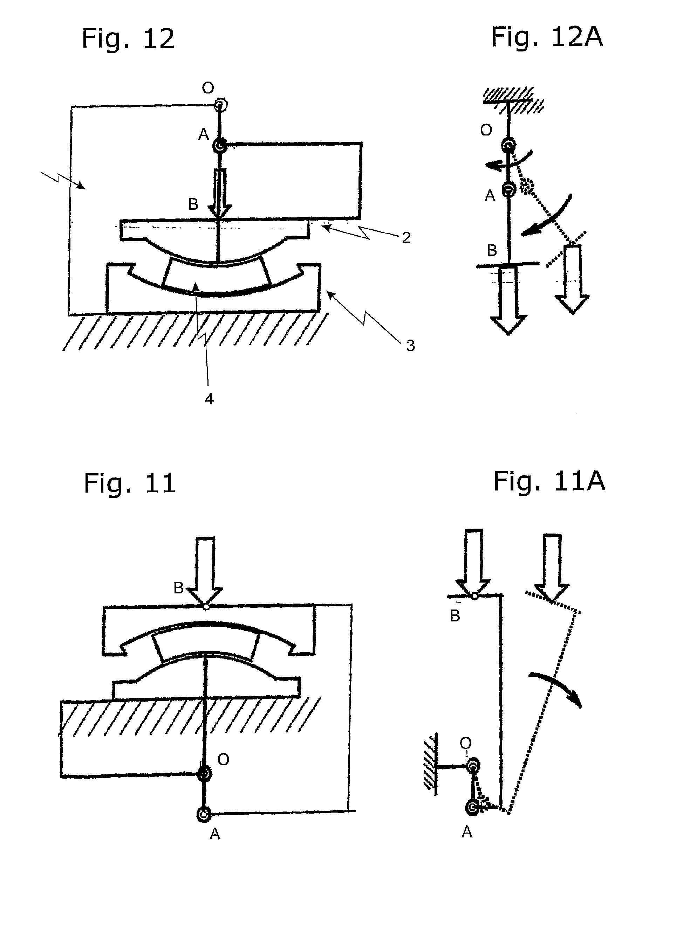

Toutefois ces prothèses connues ne répondent pas de manière satisfaisante aux contraintes évoquées, notamment en ce qu'elles ne sont pas stables en situation de compression physiologique. Ce défaut sera expliqué en détail plus loin dans la description, en relation avec les figures 10, 10A, 11 et 11A.

La présente invention a donc pour but de pallier certains inconvénients de l'art antérieur en proposant un nouveau type de disque intervertébral de conception simple, qui permette de contrôler les mouvements des différentes pièces constituant ladite prothèse afin d'éviter de soumettre les articulaires postérieures à des contraintes de charge trop importantes. En outre, la prothèse selon l'invention présente une cinématique auto-centrant et auto-stabilisant les éléments prothétiques, assurant une localisation physiologique des centres instantanés de rotation.

A cet égard, la présente invention concerne une prothèse de disque intervertébral comme défini par la revendication 1. Elle comprend au moins trois pièces dont un premier plateau dit plateau supérieur comportant une face supérieure présentant un profil convexe dans le plan sagittal de la prothèse, un second plateau dit plateau inférieur comportant une face inférieure présentant un profil plan dans le plan sagittal de la prothèse, et un noyau mobile disposé entre lesdits plateaux.

Cette prothèse est remarquable en ce que le noyau mobile comporte une surface supérieure concave en contact congruent avec la surface convexe aménagée dans la surface inférieure du plateau supérieur et une surface inférieure convexe en contact congruent avec la surface concave aménagée dans la surface supérieure du plateau inférieur, chacune desdites surfaces étant déterminées par un rayon de courbure distinct, le rayon de courbure de la surface concave du noyau étant inférieur à celui de sa surface convexe, les centres desdites surfaces concave et convexe étant situés sur l'axe de symétrie radiale du noyau central et placés du même côté de la prothèse, celui du plateau supérieur.

La prothèse discale de la présente invention possède trois composants munis de surfaces concaves et convexes. Pour répondre aux contraintes physiologiques exposées ci-avant, la disposition de ces surfaces concaves et convexes est spécifique et leur inversion n'est pas envisageable, du fait des avantages considérables procurés par cette configuration spécifique, tel que cela sera détaillé plus loin.

On décrira ci-après, à titre d'exemple non limitatif, une forme d'exécution préférentielle de la prothèse selon l'invention, en référence aux dessins annexés sur lesquels :
- la figure 1 est une vue avant en perspective de la prothèse assemblée selon un angle 3/4 supérieur,
- la figure 2 est une vue arrière en perspective de la prothèse assemblée selon un angle 3/4 supérieur,
- la figure 3 est une vue avant en perspective éclatée selon un angle 3/4 supérieur,
- la figure 4 est une vue en coupe partielle dans le plan sagittal de la prothèse,
- la figure 5 est une vue schématique semblable à la figure 4 représentant une première variante de la prothèse avec des butées,
- la figure 6 est une vue schématique semblable à la figure 4 représentant une deuxième variante de la prothèse avec des butées,
- la figure 7 est une vue schématique semblable à la figure 4 représentant la compensation des déplacements des différents éléments de la prothèse en situation de flexion-extension,
- la figure 8 est une vue schématique semblable à la figure 4 représentant la compensation des déplacements des différents éléments de la prothèse en situation de translation pure,
- la figure 9 est une vue schématique semblable à la figure 4 représentant la localisation du centre instantané de rotation de la prothèse en situation de flexion ;
- les figures 10 et 10A sont respectivement une vue en coupe partielle et une vue schématique dans le plan sagittal d'une prothèse selon l'art antérieur, où le rayon de convexité du plateau inférieur est supérieur au rayon de concavité du plateau supérieur, pour la figure 10A ladite prothèse étant représentée au repos en traits pleins et sous l'effet d'une compression en pointillés ;
- les figures 11 et 11A sont respectivement une vue en coupe partielle et une vue schématique dans le plan sagittal d'une prothèse selon l'art antérieur, où le rayon de convexité du plateau inférieur est inférieur au rayon de concavité du plateau supérieur, pour la figure 11A ladite prothèse étant représentée au repos en traits pleins et sous l'effet d'une compression en pointillés ;
- les figures 12 et 12A sont respectivement une vue en coupe partielle et une vue schématique dans le plan sagittal de la prothèse selon l'invention, pour la figure 12A ladite prothèse étant représentée au repos en traits pleins et sous l'effet d'une compression en pointillés.

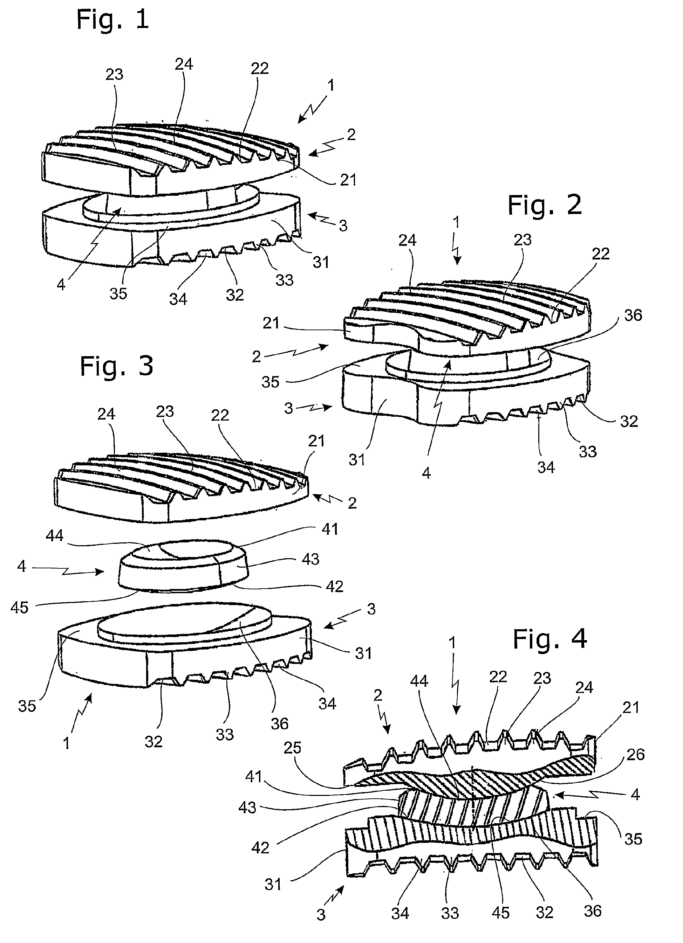

En référence aux figures 1 à 4, la prothèse 1 de disque intervertébral, selon l'invention, comprend au moins trois pièces, dont un premier plateau dit plateau supérieur 2, un second plateau dit plateau inférieur 3, et un noyau mobile 4 disposé entre les deux plateaux 2, 3, le plateau supérieur 2 et le plateau inférieur 3 s'articulant l'un par rapport à l'autre.

Ainsi, le plateau supérieur 2 comprend un corps central 21 globalement plat dont la forme et les dimensions correspondent à celles de la surface inférieure de la vertèbre du rachis cervical située au-dessus dudit plateau supérieur 2. Afin de s'adapter à ladite vertèbre et de permettre l'ancrage dudit plateau supérieur 2 au niveau osseux, la face supérieure 22 dudit corps central 21 présente un profil convexe selon le plan sagittal, légèrement bombé. De préférence, le profil selon le plan frontal de cette face supérieure 22 est également convexe. Le profil dans le plan frontal pourra également être globalement plan : l'Homme de l'art adaptera le profil de la face supérieure 22 externe du plateau supérieur 2 pour qu'il épouse le contour anatomique de la surface articulaire associée, en fonction du positionnement le long de la colonne vertébrale.

Par ailleurs, cette face supérieure 22 est munie de moyens d'ancrage 23. Dans une mode de réalisation préférentiel, les moyens d'ancrage 23 sont des dents 24 en saillie issues perpendiculairement de ladite face supérieure 22, sensiblement parallèles entre elles, perpendiculaires au plan sagittal de la prothèse 1. La section de ces dents 24 a une forme générale de trapèze régulier. Le plateau supérieur 2 comporte également sur au moins une partie de la face inférieure 25 dudit corps central 21 une surface convexe 26, avantageusement en forme de calotte sphérique disposée au voisinage du milieu de ladite face inférieure 25.

De façon analogue, le plateau inférieur 3 comprend un corps central 31 globalement plat dont la forme et les dimensions correspondent à celles de la surface supérieure de la vertèbre du rachis cervical située sous ledit plateau inférieur 3. Afin de bien s'adapter à ladite vertèbre sous-jacente et de permettre l'ancrage dudit plateau inférieur 3 avec le milieu osseux, la face inférieure 32 dudit corps central 31 présente un profil sagittal plan. De préférence, le profil de la face inférieure 32 dans le plan frontal est également plan. Le profil dans le plan frontal pourra également être convexe, avec un léger bombement : l'Homme de l'art adaptera le profil de la face inférieure 32 externe du plateau inférieur 3 pour qu'il épouse le contour anatomique de la surface articulaire associée, en fonction du positionnement le long de la colonne vertébrale.

Par ailleurs, cette face inférieure 32 externe est munie de moyens d'ancrage 33. Dans un mode de réalisation préférentiel, les moyens d'ancrage 33 sont des dents 34 semblables aux dents 24 du plateau supérieur 2 décrites précédemment, implantées perpendiculairement au plan sagittal de la prothèse 1. Le plateau inférieur 3 comporte également sur au moins une partie de la face supérieure 35 dudit corps central 31 une surface concave 36, avantageusement en forme de calotte sphérique disposée au voisinage du milieu de ladite face supérieure 35.

Pour améliorer le contact et la prise dans l'os des dents 24, 34, on pourra, par exemple, avoir une interface de type hydroxyapathite.

Le noyau mobile 4 comporte une face supérieure 41, une face inférieure 42 et une face périphérique 43 qui relie entre elles les faces supérieure et inférieure 41, 42. Dans une mode de réalisation préférentiel, cette face périphérique 43 est de forme globalement tronconique.

Pour obtenir une articulation entre le plateau supérieur 2 et le plateau inférieur 3 autour du noyau mobile 4, on comprend que le plateau supérieur 2, le plateau inférieur 3 et le noyau mobile 4 doivent être tels que la face supérieure 41 du noyau mobile 4 est en contact congruent avec la surface convexe 26 de la face inférieure 25 du plateau supérieur 2 et que la face inférieure 42 du noyau mobile 4 est en contact congruent avec la surface concave 36 de la face supérieure 35 du plateau inférieur 3. Une telle configuration permet d'avoir un déplacement relatif entre les plateaux supérieur et inférieur 2,3 et le noyau mobile 4.

Pour ce faire, le noyau mobile 4 comporte, d'une part, sur au moins une partie de sa face supérieure 41, une surface concave 44 sphérique de rayon de courbure sensiblement égal à celui de la surface convexe 26 de la surface inférieure 25 du plateau supérieur 2, et, d'autre part, sur au moins une partie de sa face inférieure 41, une surface convexe 45 sphérique de rayon de courbure sensiblement égal à celui de la surface concave 36 de la surface supérieure 35 du plateau inférieur 3.

Dans une mode de réalisation préférentiel, la surface concave 44 et la surface convexe 45 couvrent respectivement la totalité de la face supérieure 41 et de la face inférieure 42 du noyau mobile 4.

En outre, le noyau mobile 4 possède de préférence un axe de symétrie radiale de manière à avoir les centres des rayons de courbure de ses faces supérieure et inférieure 41, 42 situés sur ledit axe de symétrie et placés du même côté de la prothèse, à savoir celui du plateau supérieur 2.

Enfin, le rayon de courbure de la face inférieure 42 est supérieur à celui de la face supérieure 41 et la distance entre les centres des rayons de courbure, qui est conditionnée par l'espace intervertébral, est avantageusement la plus réduite possible.

L'Homme de l'Art n'aura aucune difficulté à dimensionner lesdits rayons de courbures et par conséquent à obtenir des vitesses de déplacement relatives des différents éléments constitutifs de la prothèse 1, permettant à ladite prothèse 1 d'être auto-adaptative et auto-stable. En outre, on comprend bien que lesdits rayons peuvent être variables en fonction de la position de la prothèse 1 le long de la colonne vertébrale, car les vitesses de déplacement relatives sont elles aussi fonction de ladite position.

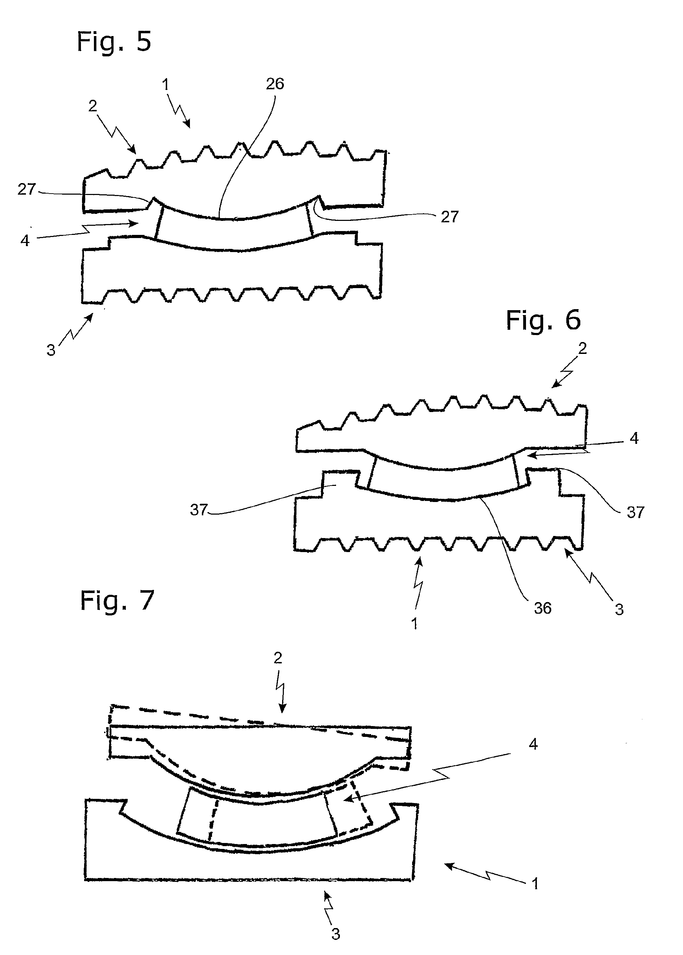

Selon une première variante de réalisation et en référence à la figure 5, pour limiter le risque d'éjection d'au moins une partie de la prothèse 1 et le débattement d'un plateau par rapport à l'autre, le plateau supérieur 2 comporte, en périphérie de sa surface convexe 26 sur sa face inférieure 25, des butées dans au moins les deux directions latéro-latérale et antéro-postérieure par rapport au positionnement de la prothèse 1 sur la colonne vertébrale. Ces butées sont avantageusement obtenues grâce à une gorge 27 réalisée dans la face inférieure 25 autour de sa surface convexe 26 sphérique.

De même, en référence à la figure 6, selon une deuxième variante de réalisation, le plateau inférieur 3 comporte, en périphérie de sa surface concave 36 sur sa face supérieure 35, des butées dans au moins les deux directions latéro-latérale et antéro-postérieure par rapport au positionnement de la prothèse 1 sur la colonne vertébrale. Ces butées sont avantageusement obtenues par un rebord périphérique 37 réalisé sur la face supérieure 35 autour de sa surface concave 36 sphérique.

La configuration particulière de la prothèse permet l'auto-centrage du noyau mobile 4 et l'auto-adaptation de la prothèse 1 afin de respecter la cinématique physiologique naturelle du rachis cervical.

En effet, en référence aux figures 7 et 8, représentant respectivement la prothèse 1 subissant une déformation de flexion-extension et de translation pure, lors du mouvement du rachis cervical et sous l'action des efforts transmis par les vertèbres adjacentes, la prothèse 1 passe, par exemple, d'une position de repos, représentée en trait fort sur la figure, à une position de travail, représentée en trait pointillé sur la figure. On comprend alors bien que le noyau mobile 4, en se déplaçant, va compenser le déplacement du plateau supérieur 2 par rapport au plateau inférieur 3. La prothèse 1, selon l'invention, permet ainsi un contrôle et une limitation des efforts exercés sur les articulaires antérieurs et postérieurs en évitant alors les problèmes d'hyperpression.

En outre, en référence à la figure 9, les centres de rotation instantanés (CIR) de l'ensemble prothétique inséré dans l'articulation intervertébrale sont de ce fait localisés dans le nuage physiologique, qu'il s'agisse de flexion extension (cas de la figure 9) ou d'inflexion latérale (non illustré).

En référence à la figure 9, la détermination du CIR est réalisée de la manière suivante : A et B sont 2 points appartenant au plateau supérieur. Le C.I.R. du plateau supérieur se situe à l'intersection des perpendiculaires aux vitesses de ces deux points (par rapport au plateau inférieur). On désignera par la suite le noyau par la lettre n, le plateau inférieur par les lettres pi et le plateau supérieur par les lettres ps.

La vitesse de A/plateau inférieur (V(A/pi)) est perpendiculaire à OA, le C.I.R. se trouve donc sur OA.

La vitesse de B / plateau inférieur est composée de la vitesse d'entraînement Ve(B) =V(A/ pi) = OA x ω(n / pi) et de la vitesse relative perpendiculaire à AB, Vr(B) = AB x ω (ps / pi).
On a donc V(B / pi) = Ve(B) + Vr(B).

Le C.I.R est à l'intersection de OA et de la perpendiculaire en B à V(B/pi)

Bien que dans cette configuration les centres de rotation des composants mécaniques soient au dessus du plateau supérieur il y a cohérence entre les CIR prothétiques et ceux de l'Unité Fonctionnelle Vertébrale.

Les figures 10,10A et 11,11A démontrent l'instabilité physiologique globale des prothèses de l'art antérieur connu, divulguée dans les documents WO 2005/094737, WO 2006/105603, US2007/270958 ou US 2004/138753, que ce soit avec un rayon de courbure de la convexité du plateau inférieur plus grand (figures 11,11A) ou plus petit (figures 10,10A) que le rayon de courbure de la concavité du plateau supérieur.

Le noyau se déplace autour du centre 0 de la convexité du plateau inférieur et le plateau supérieur se déplace autour du centre A de la convexité de la surface supérieure du noyau.

Dans le cas de la disposition des figures 10, 10A, lors du chargement du plateau supérieur en son milieu B, le centre A se déplace autour et au dessus du centre O et le point B se déplace autour et au dessus du centré A et rien n'empêche la poursuite du déplacement du point B autour du centre A et du centre A autour du centre 0 sans qu'ils puissent revenir spontanément à leur position d'origine. Seules les actions musculaires permettent de contrer cette instabilité, entrainant alors une surcharge articulaire dommageable et une dépense énergétique.

Dans le cas de la disposition des figures 11, 11A, lors du chargement du plateau supérieur en son milieu B, le centre A se déplace autour et au dessous du centre 0 et le point B se déplace autour et au dessus du centre A et rien n'empêche la poursuite du déplacement du point B autour du centre A sans qu'il puisse revenir spontanément à sa position d'origine. Seules les actions musculaires permettent de contrer cette instabilité, entrainant alors une surcharge articulaire dommageable et une dépense énergétique.

Cette instabilité des prothèses connues se retrouve également dans le concept des prothèses à noyau bi-convexe ou biconcave.

En référence aux figures 12 et 12A, on montrera maintenant comment au contraire des prothèses connues, la prothèse selon l'invention est dynamiquement stable.

Dans la prothèse selon l'invention, les surfaces concave-convexe du noyau mobile sont inversées par rapport à l'art antérieur connu : le plateau supérieur présente un surface sphérique convexe, le plateau inférieur présente une surface sphérique concave, et le noyau mobile en forme de lentille présente des surfaces sphériques concave en partie supérieure - convexe en partie inférieure.

Le rayon de courbure r de la surface supérieure concave du noyau, congruente avec la surface convexe du plateau supérieur, est plus petit que le rayon de courbure R de sa surface inférieure convexe, elle-même congruente avec la surface concave du plateau inférieur. L'épaisseur du noyau est « e ».

Dans cette configuration (voir figure 12A) le noyau se déplace toujours autour du centre 0 de la concavité du plateau inférieur, centre O situé au dessus du noyau et le centre A de la concavité du noyau se déplace sur une portion de surface sphérique de centre O et de rayon OA= R - (r+e). Lors du chargement du plateau supérieur en son milieu B, le centre A se déplace autour et au dessous du centre O, le point B se déplace autour et au dessous du centre A, le système ne s'affaisse pas et le centre A et le point B peuvent revenir spontanément à leur position d'origine, sans surcharge articulaire et sans dépense énergétique.

Ce concept positionnant le centre de rotation 0 du noyau par rapport au plateau inférieur et le centre de rotation A du plateau supérieur par rapport au noyau au dessus du plateau supérieur, avec A au dessous de 0, assure la stabilité physiologique de l'ensemble prothétique.

Selon une autre caractéristique de l'invention, les plateaux supérieur et inférieur 2,3 et le noyau mobile 4 sont avantageusement réalisés à partir de matériaux non métalliques pour permettre de réaliser des IRM afin de contrôler notamment la moelle épinière. Ainsi, de préférence, les plateaux supérieur et inférieur 2,3 sont en polyetheretherketone et le noyau mobile 4 en céramique. Ces deux matériaux ont également pour avantage d'avoir entre eux un coefficient de frottement faible permettant un glissement facile des pièces les unes contre les autres et ainsi une bonne articulation des plateaux supérieur et inférieur 2,3 sur le noyau mobile 4.

L'Homme de l'Art n'aura aucune difficulté à dimensionner les différents éléments constitutifs de la prothèse 1 selon l'invention, en respectant notamment les épaisseurs minimales liées à la nature des matériaux utilisés. Ainsi, par exemple, pour le plateau inférieur 3, on ne descendra pas en dessous de 1,3 millimètre entre le fond de la forme concave 35 et la face inférieure 32.

En outre, on comprend que suivant la morphologie du patient et notamment l'espace intervertébral, il est nécessaire d'avoir une prothèse 1 disponible sous différentes dimensions. Pour jouer sur la hauteur totale de la prothèse 1, on modifiera l'épaisseur du noyau mobile 4 et/ou l'épaisseur du plateau supérieur 2.

Enfin, il va de soi que la présente invention n'est pas limitée à l'exemple de réalisation préférentiel et à la mise en oeuvre décrits, et elle peut être modifiée ou adaptée en fonction des besoins ou des exigences particulières, sans pour autant sortir du cadre de l'invention.

## Revendications

1. Prothèse (1) de disque intervertébral comprenant au moins trois pièces dont un premier plateau dit plateau supérieur (2) comportant une face supérieure (22) présentant un profil convexe dans le plan sagittal de la prothèse, un second plateau dit plateau inférieur (3) comportant une face inférieure (32) présentant un profil plan dans le plan sagittal de la prothèse, et un noyau mobile (4) disposé entre lesdits plateaux (2,3), le noyau mobile (4) comportant une surface supérieure concave (44) en contact congruent avec la surface convexe (26) aménagée dans la surface inférieure (25) du plateau supérieur (2) et une surface inférieure convexe (45) en contact congruent avec la surface concave (36) aménagée dans la surface supérieure (35) du plateau inférieur (3), chacune desdites surfaces étant déterminées par un rayon de courbure distinct, le rayon de courbure de la surface concave (44) du noyau étant inférieur à celui de sa surface convexe (45), les centres desdites surfaces concave (44) et convexe (45) étant situés sur l'axe de symétrie radiale du noyau central et placés du même côté de la prothèse (1), celui du plateau supérieur (2) **caractérisé en ce que** le plateau inférieur (3) comporte des butées dans au moins les deux directions latéro- latérale et antéro-postérieure, ces butées sont obtenues par un rebord périphérique (37) réalisé sur la face supérieure (35) dudit plateau inférieur (3) autour de la surface concave (36) ou **en ce que** le plateau supérieur (2) comporte des butées dans au moins les deux directions latéro-latérale et antéro-postérieure, les butées sont obtenues grâce à une gorge (27) réalisée dans la face inférieure (25) autour de sa surface convexe (26).

2. Prothèse (1) de disque intervertébral, selon la revendication 1, **caractérisée en ce que** le plateau supérieur (2) comprend un corps central (21) qui comporte sur sa face supérieure (22) des moyens d'ancrage (23) et au voisinage du milieu de sa face inférieure (25) une surface convexe (26) en forme de calotte sphérique dont le rayon de courbure est sensiblement égal à celui de la surface concave (44) du noyau mobile (4), les moyens d'ancrage (23) sont des dents trapézoïdales (24) parallèles entre elles, perpendiculaires au plan sagittal de ladite prothèse (1).

3. Prothèse (1) de disque intervertébral, selon l'une quelconque des revendications précédentes, **caractérisée en ce que** le plateau inférieur (3) comprend un corps central (31) qui comporte sur sa face inférieure (32) des moyens d'ancrage (33) et, au voisinage du milieu de sa face supérieure (35), une surface concave (36) en forme de calotte sphérique dont le rayon de courbure est sensiblement égal à celui de la surface convexe (45) du noyau mobile (4), les moyens d'ancrage (33) sont des dents trapézoïdales (34) parallèles entre elles, perpendiculaires au plan sagittal de ladite prothèse (1).

4. Prothèse (1) de disque intervertébral, selon l'une quelconque des revendications précédentes, **caractérisée en ce que** le noyau mobile (4) comporte une face périphérique (43) de forme globalement tronconique.

5. Prothèse (1) de disque intervertébral, selon l'une quelconque des revendications précédentes, caractérisée en ce le plateau inférieur (3) comporte un rebord périphérique (37) réalisé sur la face supérieure (35) autour de la surface concave (36), le noyau mobile (4) comportant une face périphérique (43) de forme globalement tronconique, la face périphérique tronconique (43) venant en butée contre le rebord périphérique (37) lors du déplacement du noyau (4) par rapport au plateau inférieur (3).

6. Prothèse (1) de disque intervertébral, selon l'une quelconque des revendications précédentes, caractérisée en ce le plateau supérieur (2) comporte une gorge (27) réalisée dans la face inférieure (25) autour de sa surface convexe (26), le noyau mobile (4) comportant une face périphérique (43) de forme globalement tronconique, la face périphérique tronconique (43) venant en butée contre la gorge (27) lors du déplacement du noyau (4) par rapport au plateau supérieur (2).

7. Prothèse (1) de disque intervertébral, selon l'une quelconque des revendications précédentes, **caractérisée en ce que** les plateaux supérieur et inférieur (2,3) sont réalisés en polyetheretherketone.

8. Prothèse (1) de disque intervertébral, selon l'une quelconque des revendications précédentes, **caractérisée en ce que** le noyau mobile (4) est réalisé en céramique.

## Patentansprüche

1. Bandscheibenprothese (1), die mindestens drei Teile umfasst, von welchen eine erste Platte, obere Platte (2) genannt, eine obere Seite (22) aufweist, die ein konvexes Profil in der sagittalen Ebene der Prothese aufweist, eine zweite Platte, untere Platte (3) genannt, die eine untere Seite (32) umfasst, die ein flaches Profil in der sagittalen Ebene der Prothese aufweist, und einen beweglichen Kern (4), der zwischen den Platten (2, 3) angeordnet ist, wobei der bewegliche Kern (4) eine obere konkave Oberfläche (44) in kongruenter Berührung mit der konvexen Oberfläche (26), die in der unteren Oberfläche (25) der oberen Platte (2) eingerichtet ist, umfasst, und eine untere konvexe Fläche (45) in kongruenter Berührung mit der konkaven Oberfläche (36), die in der oberen Oberfläche (35) der unteren Platte (3) eingerichtet ist, wobei jede der Oberflächen durch einen unterschiedlichen Krümmungsradius bestimmt ist, wobei der Krümmungsradius der konkaven Oberfläche (44) des Kerns kleiner ist als der seiner konvexen Oberfläche (45), wobei die Mitten der konkaven Oberfläche (44) und konvexen Oberfläche (45) auf der radialen Symmetrieachse des zentralen Kerns liegen und auf derselben Seite der Prothese (1) platziert sind, die der oberen Platte (2) **dadurch gekennzeichnet, dass** die untere Platte (3) Anschläge in mindestens die zwei Richtungen latero-lateral und antero-posterior umfasst, wobei diese Anschläge durch einen umfänglichen Wulst (37) erzielt sind, der auf der oberen Seite (35) der unteren Platte (3) um die konkave Oberfläche (36) hergestellt ist, oder dass die obere Platte (2) Anschläge in mindestens die zwei Richtungen latero-lateral und antero-posterior umfasst, wobei die Anschläge dank einer Hohlkehle (27) erzielt werden, die in der unteren Seite (25) ihrer konvexen Oberfläche (26) hergestellt ist.

2. Bandscheibenprothese (1) nach Anspruch 1, **dadurch gekennzeichnet, dass** die obere Platte (2) einen zentralen Körper (21) umfasst, der auf seiner oberen Seite (22) Verankerungsmittel (23) und in der Nähe der Mitte seiner unteren Seite (25) eine konvexe Oberfläche (26) in Form einer sphärischen Kalotte, deren Krümmungsradius im Wesentlichen gleich dem der konkaven Oberfläche (44) des beweglichen Kerns (4) ist, umfasst, wobei die Verankerungsmittel (23) trapezförmige Zähne (34), die zueinander parallel sind, senkrecht zu der sagittalen Ebene der Prothese (1) sind.

3. Bandscheibenprothese (1) nach einem der vorhergehenden Ansprüche, **dadurch gekennzeichnet, dass** die untere Platte (3) einen zentralen Körper (31) umfasst, der auf seiner unteren Seite (32) Verankerungsmittel (33) umfasst, und, in der Nähe der Mitte seiner oberen Seite (35), eine konkave Oberfläche (36) in Form einer sphärischen Kalotte, deren Krümmungsradius im Wesentlichen gleich dem der konvexen Oberfläche (45) des beweglichen Kerns (4) ist, wobei die Verankerungsmittel (33) trapezförmige Zähne (34), die zueinander parallel sind, senkrecht zu der sagittalen Ebene der Prothese (1) sind.

4. Bandscheibenprothese (1) nach einem der vorhergehenden Ansprüche, **dadurch gekennzeichnet, dass** der bewegliche Kern (4) eine umfängliche Seite (43) mit insgesamt kegelstumpfförmiger Form umfasst.

5. Bandscheibenprothese (1) nach einem der vorhergehenden Ansprüche, **dadurch gekennzeichnet, dass** die untere Platte (3) einen umfänglichen Wulst (37) umfasst, der auf der oberen Seite (35) um die konkave Oberfläche (36) hergestellt ist, wobei der bewegliche Kern (4) eine umfängliche Seite (43) mit insgesamt kegelstumpfförmiger Form umfasst, wobei die periphere kegelstumpfförmige Seite (43) beim Bewegen des Kerns (4) in Bezug auf die untere Platte (3) zum Anschlagen gegen den umfänglichen Wulst (37) kommt.

6. Bandscheibenprothese (1) nach einem der vorhergehenden Ansprüche, **dadurch gekennzeichnet, dass** diese obere Platte (2) eine Hohlkehle (27) umfasst, die in der unteren Seite (25) um ihre konvexe Oberfläche (26) hergestellt ist, wobei der bewegliche Kern (4) eine umfängliche Seite (43) mit allgemein kegelstumpfförmiger Form umfasst, wobei die kegelstumpfförmige umfängliche Seite (43) bei der Bewegung des Kerns (4) in Bezug auf die obere Platte (2) zum Anschlagen gegen die Hohlkehle (27) kommt.

7. Bandscheibenprothese (1) nach einem der vorhergehenden Ansprüche, **dadurch gekennzeichnet, dass** die obere und untere Platte (2, 3) aus Polyetheretherketon hergestellt sind.

8. Bandscheibenprothese (1) nach einem der vorhergehenden Ansprüche, **dadurch gekennzeichnet, dass** der bewegliche Kern (4) aus Keramik hergestellt ist.

## Claims

1. Intervertebral disc prosthesis (1) comprising at least three components, namely a first plate, called the upper plate (2), comprising an upper face (22) having a convex profile in the sagittal plane of the prosthesis, a second plate, called the lower plate (3), comprising a lower face (32) having a plane profile in the sagittal plane of the prosthesis, and a movable core (4) arranged between said plates (2, 3), the movable core (4) comprising a concave upper surface (44) in congruent contact with the convex surface (26) provided in the lower surface (25) of the upper plate (2) and a convex lower surface (45) in congruent contact with the concave surface (36) provided in the upper surface (35) of the lower plate (3), each of said surfaces being defined by a distinct radius of curvature, the radius of curvature of the concave surface (44) of the core being less than that of the convex surface (45) thereof, the centres of said concave (44) and convex (45) surfaces being situated on the radial axis of symmetry of the central core and being placed on the same side of the prosthesis (1), namely that of the upper plate (2), **characterised in that** the lower plate (3) comprises abutments in at least both the latero-lateral and antero-posterior directions, these abutments are obtained by means of a peripheral edge (37) produced on the upper face (35) of said lower plate (3) about the spherical concave surface (36) or **in that** the upper plate (2) comprises abutments in at least both the latero-lateral and antero-posterior directions, the abutments are obtained by means of a groove (27) produced in the lower face (25) about the convex surface (26) thereof.

2. Intervertebral disc prosthesis (1) according to claim 1, **characterised in that** the upper plate (2) comprises a central body (21) comprising on the upper face (22) thereof anchoring means (23) and in the vicinity of the middle of the lower face (25) thereof a convex surface (26) in the form of a spherical cap wherein the radius of curvature is substantially equal to that of the concave surface (44) of the movable core (4), the anchoring means (23) are trapezoidal teeth (24) that are parallel with each other, perpendicular to the sagittal plane of said prosthesis (1).

3. Intervertebral disc prosthesis (1) according to any of the above claims, **characterised in that** the lower plate (3) comprises a central body (31) comprising on the lower face (32) thereof anchoring means (33) and in the vicinity of the middle of the lower face (35) thereof a convex surface (36) in the form of a spherical cap wherein the radius of curvature is substantially equal to that of the concave surface (45) of the movable core (4), the anchoring means (33) are trapezoidal teeth (34) that are parallel with each other, perpendicular to the sagittal plane of said prosthesis (1).

4. Intervertebral disc prosthesis (1) according to any of the above claims, **characterised in that** the movable core (4) comprises a peripheral face (43) with an overall tapered shape.

5. Intervertebral disc prosthesis (1) according to any of the above claims, **characterised in that** the lower plate (3) comprises a peripheral edge (37) produced on the upper face (35) about the concave surface (36), the movable core (4) comprising a peripheral face (43) with an overall tapered shape, the tapered peripheral face (43) abutting against the peripheral edge (37) during the movement of the core (4) in relation to the lower plate (3).

6. Intervertebral disc prosthesis (1) according to any of the above claims, **characterised in that** the upper plate (2) comprises a groove (27) produced in the lower face (25) about the convex surface (26) thereof, the movable core (4) comprising a peripheral face (43) with an overall tapered shape, the tapered peripheral face (43) abutting against the groove (27) during the movement of the core (4) in relation to the upper plate (2).

7. Intervertebral disc prosthesis (1) according to any of the above claims, **characterised in that** the upper and lower plates (2, 3) are made of polyetheretherketone.

8. Intervertebral disc prosthesis (1) according to any of the above claims, **characterised in that** the movable core (4) is made of ceramic.
